# EUROPÄISCHE PATENTSCHRIFT

(11) **EP 1 221 891 B1**
(45) Veröffentlichungstag und Bekanntmachung des Hinweises auf die Patenterteilung: **15.09.2004**
(21) Anmeldenummer: 00945553.6
(22) Anmeldetag: 22.05.2000
(51) Int. Cl.: A61B 5/00

(54) **VERFAHREN UND GERÄT ZUR ÜBERTRAGUNG PERSONENBEZOGENER DATEN**
METHOD AND DEVICE FOR TRANSMITTING PERSON-RELATED DATA
PROCEDE ET DISPOSITIF DE TRANSMISSION DE DONNEES RELATIVES A UNE PERSONNE

(30) Priorität: 20.10.1999 DE 19950578
(43) Veröffentlichungstag der Anmeldung: 17.07.2002
(73) Patentinhaber: SIEMENS AKTIENGESELLSCHAFT, 80333 München (DE)
(72) Erfinder: KINZEL, Winfried, D-81737 München (DE); BROMBA, Manfred, D-81379 München (DE); PETER, Martin, D-80333 München (DE)
(86) Internationale Anmeldenummer: PCT/DE2000/001638
(87) Internationale Veröffentlichungsnummer: WO 2001/028413

(56) Entgegenhaltungen:
- WO-A-97/08868
- WO-A-97/40745
- DE-A- 3 943 097
- DE-A- 19 541 672
- US-A- 5 544 661
- US-A- 5 577 510

## Beschreibung

Die Erfindung betrifft ein Verfahren zur Übertragung personenbezogener Daten und ein Gerät zur Durchführung dieses Verfahrens.

Ein Verfahren nach dem Oberbegriff von Anspruch 1 ist aus DE-A-39 43 097 bekannt.

In vielen Situationen, insbesondere in Notfällen, ist es äußerst wünschenswert oder sogar unumgänglich, möglichst schnell und umfassend zu einem bestimmten Menschen personenbezogene Daten, beispielsweise medizinische Daten wie Blutgruppe, Impfdaten, Medikamentenunverträglichkeiten etc., zu erhalten. Da eine falsche Zuordnung von Daten zu der Person lebensentscheidende Auswirkungen haben kann, ist es unbedingt notwendig, daß Verwechslungen hierbei vermieden werden.

Bisher sind in Notfällen beispielsweise das menschliche Gedächtnis, medizinische Ausweise, wie Impfpässe, Bluteroder Diabetikerausweise, Kennmarken, Karteien oder ähnliches erforderlich, aus denen die notwendigen Daten hervorgehen. Diese Datenträger sind oftmals verstreut aufbewahrt, in Notfällen auch nicht unbedingt ohne weiteres zu finden. Dies betrifft insbesondere Fälle, wo die Person selber, beispielsweise aufgrund von Bewußtlosigkeit, nicht ansprechbar ist oder aber wo es sich um eine verwirrte, hilfsbedürftige Person handelt.

Es ist Aufgabe der vorliegenden Erfindung, ein Verfahren zur Übertragung personenbezogener Daten, insbesondere medizinischer Daten, zu schaffen, mit dem es möglich ist, kurzfristig und verwechslungsfrei Daten über eine bestimmte Person zu erhalten.

Es ist weiterhin Aufgabe, ein Gerät zur Durchführung des Verfahrens zu schaffen.

Diese Aufgaben werden durch ein Verfahren gemäß dem Anspruch 1 bzw. ein Gerät gemäß Anspruch 13 gelöst.

Bei dem erfindungsgemäßen Verfahren werden mit einer Biometrieerfassungseinrichtung von einer Person biometrische Daten erfaßt. Diese die Person eindeutig identifizierenden biometrischen Daten werden an eine zentrale Speichereinrichtung gesandt, in der die personenbezogenen Daten gespeichert sind. Anhand der biometrischen Daten werden in der Speichereinrichtung die personenbezogenen Daten zu der Person automatisch selektiert, indem die von der Biometrieerfassungseinrichtung erhaltenen biometrischen Daten mit entsprechenden Datensätzen verglichen werden, die den personenbezogenen Daten zugeordnet sind.

Die personenbezogenen Daten werden dann anschließend sofort an eine am Ort der Biometrieerfassungseinrichtung befindliche Empfangseinrichtung gesandt. Sie können dort weiterverarbeitet oder sofort ausgegeben werden.

Ein erfindungsgemäßes Gerät weist dementsprechend eine Biometrieerfassungseinrichtung zur Erfassung biometrischer Daten einer Person, eine Sendeeinrichtung zum Übertragen der biometrischen Daten, eine Empfangseinrichtung zum Empfang der personenbezogenen Daten und eine Einrichtung zur Ausgabe und/oder Weiterverarbeitung dieser Daten, beispielsweise ein Display, einen Drucker oder zur akustischen Ausgabe einen Lautsprecher auf.

Die Biometrieerfassungseinrichtung und die Empfangseinrichtung sind hierbei vorzugsweise als mobile Geräte ausgebildet, so daß sie, beispielsweise von einem Unfallarzt, an einen beliebigen Ort mitgenommen werden können. Selbstverständlich ist es auch möglich, daß die Biometrieerfassungseinrichtung und die Empfangseinrichtung stationäre Geräte, z. B. in der Unfallaufnahme eines Krankenhauses, sind.

Bei einem besonders bevorzugten Ausführungsbeispiel sind die Biometrieerfassungseinrichtung und die Empfangseinrichtung gemeinsam in einem mobilen Gerät angeordnet. Bei diesem mobilen Gerät kann es sich beispielsweise um eine Art PDA, Organizer oder ähnliches handeln, welches mit entsprechenden Sende- und Empfangsanlagen und einer Biometrieerfassungseinrichtung ausgestattet ist. Eine weitere bevorzugte Möglichkeit besteht darin, daß das Gerät ein Mobiltelefon mit einer Biometrieerfassungseinrichtung ist. Ein derartiges Mobilfunkgerät weist im Prinzip schon alle nötigen Sende- und Empfangsanlagen sowie Einrichtungen zur Weiterverarbeitung und Ausgabe der Informationen auf. Die Biometrieerfassungseinrichtung kann dabei entweder in das Mobiltelefon integriert sein oder, quasi als Zusatzgerät, über eine Schnittstelle mit einem "normalen" Mobiltelefon gekoppelt sein.

Bei den personenbezogenen Daten kann es sich prinzipiell um beliebige Arten von Daten, beispielsweise um einen Namen oder eine Adresse, handeln. Vorzugsweise handelt es sich jedoch, wie bereits oben erwähnt, um medizinische Daten wie spezielle Krankheiten, Blutgruppen, Impfdaten, Medikamentenunverträglichkeiten, Allergien oder eine vollständige Krankenvorgeschichte.

Insbesondere erlaubt es das Verfahren auch, daß ohne eine direkte namensmäßige Identifizierung im Falle eines Notfalls medizinisch wichtige Daten direkt den biometischen Daten zugeordnet sind. Dies bedeutet, daß lediglich die in einem Notfall relevanten medizinischen Daten, wie beipielsweise die Blutgruppe, Medikamentenunverträglichkeit, Impfdaten o. ä. abgefragt werden können, wobei diese Daten in der Speichereinrichtung nicht mit dem Namen und der Adresse der Person verbunden sind. Somit können unter dem Namen und der Adresse auch diese personenrelevanten Daten nicht abgefragt werden. Eine Abfrage ist dann folglich nur durch eine direkte Erfassung der biometrischen Daten am Körper der Person möglich, wozu diese in der Regel die Zustimmung geben muß, es sei denn, sie ist nicht mehr ansprechbar. Außer in derartigen extremen Ausnahmefällen ist somit ohne Wissen der Person keine Abfrage der Daten möglich.

Bei den biometrischen Daten kann es sich prinzipiell um beliebige Datensätze handeln, die eine eindeutige Identifizierung der Person ermöglichen. Hierfür bietet sich insbesondere eine Fingerabdruckaufnahme oder eine Irismusteraufnahme der Person an. Es kann sich beispielsweise aber auch um Daten einer Sprachanalyse oder einer DNA-Analyse handeln.

Die Biometrieerfassungseinrichtung weist entsprechend der jeweiligen zu erfassenden biometrischen Daten eine optische Aufnahmeeinheit, beispielsweise eine Kamera, zur Aufnahme des Fingerabdrucks oder des Irismusters, auf. Im Falle einer Irismustererkennung weist diese Biometrieerfassungseinrichtung zusätzlich eine Beleuchtungseinrichtung auf, mit der die Iris während der Aufnahme beleuchtet wird.

Zur Erfassung von Fingerabdrücken bietet sich auch an, eine kapazitive Aufnahmeeinheit zu verwenden. Derartige kapazitive Fingerabdruck-Erfassungseinheiten sind inzwischen als fertige Module relativ kostengünstig zu erhalten.

Die empfangenen biometrischen Daten werden in der Speichereinrichtung mit den hinterlegten biometrischen Datensätzen verglichen, die den verschiedenen personenbezogenen Daten zugeordnet sind. Dabei wird der biometrische Datensatz, welcher mit den empfangenen biometrischen Daten die größte Übereinstimmung aufweist, als "richtiger" Datensatz erkannt. Um Fehlzuordnungen zum Beispiel bei Personen zu vermeiden, deren Daten überhaupt nicht in der Speichereinrichtung hinterlegt sind, wird zusätzlich gefordert, daß die Übereinstimmung der empfangenen biometrischen Daten mit den hinterlegten biometrischen Daten einen vorgegebenen Schwellenwert überschreitet. Die diesem "richtigen" biometrischen Datensatz zugeordneten gespeicherten personenbezogenen Daten werden dann an die Empfangseinrichtung gesendet. Zur Erhöhung der Sicherheit bei der Personenzuordnung können sich unter den abrufbaren personenbezogenen Datensätze auch solche persönlichen Merkmale, beispielsweise Alter, Geschlecht, Haarfarbe, Größe etc., befinden, die vor Ort mit der Person verglichen werden können.

Zum Vergleich werden die biometrischen Daten vorzugsweise zunächst bezüglich bestimmter Parameter bzw. Charakteristika, beispielsweise bei einem Fingerabdruck bezüglich dessen Minutien, analysiert und dann in Form der dabei ermittelten Parameterwerte zur Selektion der personenbezogenen Daten verwendet. Die den personenbezogenen Daten zugeordneten biometrischen Datensätze sind in diesem Fall entsprechende Parametersätze, so daß ein direkter Vergleich der Parameter erfolgen kann. Auf diese Weise ist eine sehr schnelle Erkennung und Zuordnung der empfangenen biometrischen Daten zu den hinterlegten biometrischen Daten möglich.

Die Verwendung solcher Parametersätze aus einer Analyse der kompletten biometrischen Daten reduziert außerdem den Speicherplatz, der zur Hinterlegung der gesamten biometrischen Datensätze benötigt wird.

Weiterhin besteht bei einer Verwendung solcher festgelegter Parametersätze die Möglichkeit, daß die Analyse der biometrischen Daten bezüglich der Parameter bereits in der Biometrieerfassungseinrichtung erfolgt und dann die ermittelten Parameterwerte an die Speichereinrichtung gesandt werden. Da diese Daten im Gegensatz zu der vollen biometrischen Aufnahme, beispielsweise das Bild eines Fingerabdrucks oder eines Irismusters, einen erheblich reduzierteren Umfang aufweisen, ist eine schnellere und einfachere Übersendung der Daten möglich.

Voraussetzung ist hierfür natürlich, daß in der zentralen Speichereinrichtung und in der Biometrieerfassungseinrichtung die exakt gleiche Analyse durchgeführt wird und die gleichen Parameter abgespeichert werden.

Solange eine entsprechende Normierung der Analyse bzw. des Erkennungsverfahrens noch nicht gegeben ist, und beispielsweise verschiedene Biometrieerfassungseinrichtungen mit unterschiedlichen Parametern arbeiten oder, z. B. in unterschiedlichen Ländern, verschiedene zentrale Speichereinrichtungen existieren, die jeweils unterschiedlich die biometrischen Daten analysieren, kann es auch sinnvoll sein, aus Gründen der Kompatibilität die biometrischen Daten vollständig, beispielsweise als komplettes Bild eines Fingerabdrucks oder einer Irismusteraufnahme, zu übersenden, und die Analyse erst in der Speichereinrichtung nach deren dortigen Standard durchzuführen.

Insbesondere bei der Übersendung eines vollständigen Bildes ist es sinnvoll, wenn zusätzlich zu den biometrischen Daten ein Skalierungsparameter von der Biometrieerfassungseinrichtung an die Speichereinrichtung gesandt wird. Dieser Skalierungsparameter gibt beispielsweise einen genau definierten Vergleichsmaßstab an, um auf diese Weise zu verhindern, daß Parameter, die von räumlichen Abmessungen abhängen, aufgrund unterschiedlicher Biometrieerfassungseinrichtungen oder unterschiedlicher Aufnahmemethoden verfälscht werden. Bei dem Vergleichsmaßstab kann es sich z. B. um einen tatsächlich im aufgenommenen Bild eingeblendeten Maßstab handeln.

Um die zu übersendenden Datensätze noch weiter zu reduzieren, können die biometrischen Daten bzw. auch die personenbezogenen Daten vor der Übersendung noch zusätzlich auf übliche Weise datentechnisch komprimiert werden.

Ebenso können die biometrischen Daten bzw. die personenbezogenen Daten zur Erhöhung der Sicherheit codiert übersendet werden.

Weiterhin kann es sinnvoll sein, wenn die zentrale Speichereinrichtung derart eingerichtet ist, daß nur von bestimmten autorisierten bzw. vertrauenswürdigen Quellen gesendete biometrische Daten akzeptiert werden. Dies läßt sich durch die Verwendung eines sogenannten "Challenge & Response"-Verfahrens absichern. Hierbei wird die zentrale Speichereinrichtung angewählt und sendet zunächst eine Zufallszahl zurück. Diese Zufallszahl wird zusammen mit dem sogenannten "Hash-Wert" der biometrischen Daten mit einem geräteeigenen Schlüssel verschlüsselt und mit den biometrischen Daten als Signatur mitgesendet. Die zentrale Speichereinrichtung entschlüsselt die Signatur und überprüft den Hash-Wert und die Zufallszahl. Wenn beide Werte stimmen, ist die Authentizität der "Quelle" der biometrischen Daten sichergestellt.

Alternativ oder zusätzlich kann, insbesondere bei der Verwendung von Mobilfunksystemen, auch die Nummer des sendenden bzw. anfragenden Geräts in der zentralen Speichereinrichtung registriert sein. Diese Registrierung wird dann bei einer Anfrage überprüft.

Die Daten können im Prinzip auf beliebige Weise übermittelt werden. Bei einem besonders bevorzugten Ausführungsbeispiel werden die biometrischen Daten und auch die personenbezogenen Daten paketweise über einen Mobilfunkkanal gesendet. Die paketweise Übertragung hat u.a. den Vorteil, daß auf einfache Weise redundante Daten eingefügt werden können. Zum anderen wird die kryptographische Verschlüsselung leichter. Beides erhöht die Sicherheit bei der Übertragung. Zudem ist solch eine Übertragung parallel zur Belegung des Sprachkanals möglich. Daher kann, wenn das Gerät, wie beispielsweise ein Mobiltelefon, eine entsprechende Sprecheinrichtung aufweist, die helfende Person das Gerät gleichzeitig zur Kommunikation mit einem Krankenhaus zur Vorbereitung auf den Patienten nutzen.

Bei dem derzeitigen GSM-Standard bietet sich an, den dort vorhandenen Standard-Signalisierungskanal zu verwenden und die Daten wie eine Kurznachricht nach dem SMS-Standard (Short Message Service) zu versenden. Der Vorteil dieses SMS-Standards ist die Übertragungszeit von wenigen Sekunden und die Absicherung über eine Empfangsbestätigung, da die Nachricht nur dann auf den Servern des GSM-Netzes gelöscht wird, wenn die Message beim Empfänger fehlerfrei angekommen ist. Außerdem wartet in der Regel der Server solange, bis die Nachricht vom Empfänger quittiert wurde. Die Benutzung dieses Standards hat außerdem den Vorteil, daß die Geräte sofort universell eingesetzt werden können, ohne daß ein neuer Standard erzeugt werden muß. Nach dem derzeitigen Standard können innerhalb einer Short Message bis zu 160 Zeichen untergebracht werden. Dies reicht in der Regel aus, um sowohl die Parameter der biometrischen Daten als auch die personenbezogenen Daten zu übertragen. Selbstverständlich ist es auch möglich, andere Standards, beispielsweise das GPRS-System(General Package Radio System) zu verwenden, welches ebenfalls auf dem Signalisierungskanal des GSM-Netzes arbeitet. Als weitere Möglichkeit bietet sich auch das HSCSD-System (High Speed Circuit Switched Data) an, bei dem die Datenübertragung paketweise auf einem Sprachkanal erfolgt.

Alle diese Verfahren zur Übertragung der Daten im Mobilfunknetz haben den Vorteil, daß derartige Netze nahezu flächendeckend zur Verfügung stehen und keinerlei neuer Netzaufbau mehr nötig ist, so daß eine schnelle Umsetzung des Verfahrens gewährleistet ist.

Die Ausstattung von Mobiltelefonen mit einer entsprechenden integrierten oder über eine Schnittstelle koppelbaren Biometrieerfassungseinrichtung hat weiterhin den Vorteil, daß derartige Geräte in relativ kurzer Zeit weit verbreitet sind und somit die Wahrscheinlichkeit, im Notfall ein entsprechendes Gerät zur Verfügung zu haben, recht hoch ist. Die Geräte und das Verfahren würden dann auch unvorbereiteten Ersthelfern zur Verfügung stehen.

Als weitere Option besteht die Möglichkeit, daß sich auch die abfragende bzw. helfende Person mittels eigener biometrischer Daten, beispielsweise eines Fingerabdrucks oder einer Irisaufnahme, ausweisen muß. Es kann dann in der Speichereinrichtung unterschieden werden, welche Daten dieser Person zugänglich gemacht werden. So könnten beispielsweise bei Abruf durch eine nicht durch Hinterlegung eines entsprechenden biometrischen Datensatzes autorisierte Person lediglich für eine Erstbehandlung lebensnotwendige Daten übermittelt werden, wogegen bei einem bestimmten Arzt, welcher in der Speichereinrichtung als solcher ausgewiesen ist, auch weitere medizinische Daten über den Patienten herausgegeben werden.

Selbstverständlich ist es auch möglich, daß dieses Verfahren nicht nur in Notfallsituationen eingesetzt wird, sondern auch standardmäßig zur Übertragung von Patientendaten in Krankenhäusern, Arztpraxen und dergleichen.

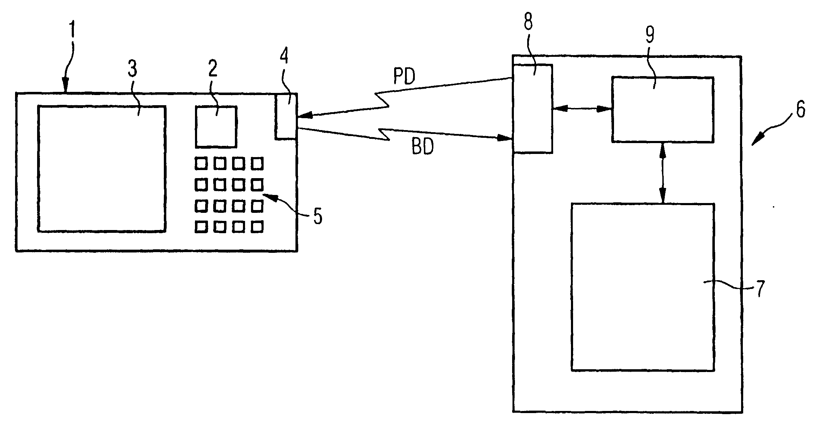

Die Erfindung wird im folgenden unter Hinweis auf die beigefügte Zeichnung anhand eines Ausführungsbeispiels näher erläutert. Die dargestellten Merkmale können nicht nur in den genannten Kombinationen, sondern auch einzeln oder in anderen Kombinationen erfindungswesentlich sein.

Die beiliegende einzige Figur zeigt hierbei ein erfindungsgemäßes mobiles Gerät 1 mit einer Biometrieerfassungseinrichtung 2 und einer kombinierten Sende- und Empfangseinrichtung 4, mit welcher die biometrischen Daten BD an die zentrale Speichereinrichtung 6 gesendet werden und mit der die personenbezogenen Daten PD von der zentralen Speichereinrichtung 6 empfangen werden. Weiterhin weist dieses mobile Gerät 1 eine Anzeigeeinrichtung in Form eines Displays 3 auf. Außerdem enthält das Gerät 1 eine Eingabeeinrichtung in Form einer Tastatur 5, mit der beispielsweise die zentrale Speichereinrichtung 6 durch Eingabe einer bestimmten Teilnehmernummer angewählt werden kann, um den Funkkanal aufzubauen.

Bei dem dargestellten Ausführungsbeispiel weist die Biometrieerfassungseinrichtung 2 eine kapazitive Aufnahmeeinheit in Form einer Sensorfläche auf. Auf diese Sensorfläche wird ein Finger der Person aufgelegt und damit die Struktur des Fingerabdrucks erfaßt. Im Gerät 1 selbst werden dann die biometrischen Daten BD hinsichtlich verschiedener Parameter analysiert. Im vorliegenden Ausführungsbeispiel werden zur Analyse der Fingerabdrücke lediglich 12 Parameter, sogenannte 12 Minutien, ermittelt. Bei diesen Parametern handelt es sich um Singularitäten bzw. Diskontinuitäten im Abbild des Fingerabdrucks. Hierbei kann es sich beispielsweise um Anfangspunkte oder Endpunkte von bestimmten Linien handeln, die besonders im Bild herausstechen. Für diese 12 Parameter wird dann jeweils die X- und Y-Position innerhalb eines Ortsrasters angegeben. Zusätzlich wird in einem Bit festgelegt, um welche Art von Diskontinuität es sich handelt. Diese Parameterwerte werden dann als Parametersatz ggf. noch komprimiert und anschließend über die Sende-/Empfangseinrichtung 4 über einen paketorientierten Mobilfunkkanal an eine Sende-/Empfangseinrichtung 8 der zentralen Speichereinrichtung 6 gesendet.

In der Speichereinrichtung 6 sind in einem Speicher 7 zu den verschiedenen Personen personenbezogene Datensätze hinterlegt. Diesen personenbezogenen Datensätzen sind jeweils biometrische Datensätze zugeordnet.

Die Abspeicherung dieser biometrischen Datensätze mit den zugehörigen Datensätzen kann beispielsweise ebenfalls über das Gerät 1 erfolgen. Hierzu kann die Person selber von allen 10 Fingern jeweils die Fingerabdrücke von der Biometrieerfassungseinrichtung 2 aufnehmen lassen und diese gemeinsam mit den über die Tastatur 5 eingegebenen personbezogenen Daten an die zentrale Speichereinrichtung 6 senden, die die Daten dort auftragsgemäß abspeichert.

Bei einem Abruf wird der übersendete Fingerabdruck mit den verschiedenen biometrischen Datensätzen des Speichers 7 in einer Fingerabdruckerkennungseinrichtung 9 verglichen. Der Vergleich erfolgt hierbei durch einen direkten Vergleich der bei einer Analyse des Fingerabdruckbildes erhaltenen, reduzierten Parametersätze. Zur eindeutigen Identifizierung wird dann der hinterlegte Parametersatz herausgesucht, der dem übersendeten Parametersatz am ähnlichsten ist. Zusätzlich wird kontrolliert, ob die Ähnlichkeit oberhalb eines vorgegebenen Schwellenwertes liegt. Wenn dies der Fall ist, gilt die Person als eindeutig identifiziert, so daß die zu dem gefundenen biometrischen Datensatz gehörigen personenbezogenen Daten PD wiederum über die Sende-/Empfangseinrichtung 8 der Speichereinrichtung 6 an die Sende-/Empfangseinrichtung 4 des Geräts 1 gesendet werden können. Die übersendeten Daten werden dann dort auf dem Display 3 angezeigt.

Zusätzlich zu den biometrischen Daten können über die Tastatur 5 auch Auswahlkriterien für die zu übertragenden Daten verschickt werden.

Ebenso ist es selbstverständlich möglich, weitere Daten oder Erkennungsmerkmale, beispielsweise Haarfarbe, Augenfarbe oder ähnliches, anzugeben. Ebenso können auf diesem Wege zusätzliche personenbezogenen Daten PD in der zentralen Speichereinrichtung 6 eingespeichert oder die hinterlegten Daten geändert werden.

## Patentansprüche

1. Verfahren zur Übertragung personenbezogener Daten (PD), bei welchem mit einer Biometrieerfassungseinrichtung (2) von einer Person biometrische Daten (BD) erfaßt werden und an eine zentrale Speichereinrichtung (6) zum Speichern der personenbezogenen Daten (PD) gesandt werden, und anhand dieser biometrischen Daten (BD) in der Speichereinrichtung (6) personenbezogene Daten (PD) zu dieser Person selektiert und diese personenbezogenen Daten (PD) an eine am Ort der Biometrieerfassungseinrichtung (2) befindliche Empfangseinrichtung (4) gesandt und dort weiterverarbeitet und/oder ausgegeben werden, wobei
die empfangenen biometrischen Daten (BD) in der Speichereinrichtung (6) mit dort hinterlegten, den personenbezogenen Daten (PD) zugeordneten biometrischen Datensätzen verglichen werden, **dadurch gekennzeichnet, daß** der biometrische Datensatz, welcher mit den empfangenen biometrischen Daten (BD) die größte Übereinstimmung aufweist und bei welchem das Maß der Übereinstimmung oberhalb eines vorgegebenen Schwellenwertes liegt, ausgewählt wird und die diesem biometrischen Datensatz zugeordneten gespeicherten personenbezogenen Daten (PD) an die Empfangseinrichtung (4) gesendet werden.

2. Verfahren nach Anspruch 1, **dadurch gekennzeichnet, daß** die Biometrieerfassungseinrichtung (2) und die Empfangseinrichtung (4) als mobile Geräte ausgebildet sind.

3. Verfahren nach Anspruch 1 oder 2, **dadurch**
**gekennzeichnet, daß** die Biometrieerfassungseinrichtung (2) und die Empfangseinrichtung (4) gemeinsam in einem mobilen Gerät (1) angeordnet sind.

4. Verfahren nach einem der Ansprüche 1 bis 3, **dadurch gekennzeichnet, daß** die personenbezogenen Daten (PD) medizinische Daten sind.

5. Verfahren nach einem der Ansprüche 1 bis 4, **dadurch**
**gekennzeichnet, daß** die biometrischen Daten (BD) eine Fingerabdruckaufnahme umfassen.

6. Verfahren nach einem der Ansprüche 1 bis 5, **dadurch gekennzeichnet, daß** die biometrischen Daten (BD) eine Irismusteraufnahme umfassen.

7. Verfahren nach einem der vorhergehenden Ansprüche, **dadurch gekennzeichnet, daß** die biometrischen Daten (BD) bezüglich bestimmter Parameter analysiert werden, und die biometrischen Daten (PD) in Form der dabei ermittelten Parameterwerte zur Selektion der personenbezogenen Daten in der Speichereinrichtung (6) verwendet werden.

8. Verfahren nach Anspruch 7 , **dadurch gekennzeichnet, daß** die Analyse der biometrischen Daten (BD) bezüglich bestimmter Parameter in der Biometrieerfassungseinrichtung (2) erfolgt, und die biometrischen Daten (BD) in Form der ermittelten Parameterwerte an die Speichereinrichtung (6) gesandt werden.

9. Verfahren nach einem der vorstehenden Ansprüche, **dadurch gekennzeichnet, daß** zusätzlich zu den biometrischen Daten (BD) ein Skalierungsparameter von der Biometrieerfassungseinrichtung (2) an die Speichereinrichtung (6) gesandt wird.

10. Verfahren nach einem der vorstehenden Ansprüche, **dadurch gekennzeichnet, daß** die biometrischen Daten (BD) und/oder die personenbezogenen Daten (PD) vor der Übersendung komprimiert werden.

11. Verfahren nach einem der vorstehenden Ansprüche, **dadurch gekennzeichnet, daß** die biometrischen Daten (BD) und/oder die personenbezogenen Daten (PD) codiert übersendet werden.

12. Verfahren nach einem der vorstehenden Ansprüche, **dadurch gekennzeichnet, daß** die biometrischen Daten (BD) und/oder die personenbezogenen Daten (PD) paketweise über einen Mobilfunkkanal gesendet werden.

13. System zur Durchführung des Verfahrens gemäß einem der vorstehenden Ansprüche, umfassend
- ein Gerät mit einer Biometrieerfassungseinrichtung (2) zur Erfassung biometrischer Daten (BD) einer Person, einer Sendeeinrichtung (4) zum Übertragen der biometrischen Daten, einer Empfangseinrichtung (4) zum Empfang der personenbezogenen Daten (PD) und einer Einrichtung (3) zur Ausgabe und/oder Weiterverarbeitung dieser Daten (PD), und
- eine Speichereinrichtung (6) zum Speichern der personenbezogenen Daten (PD) der Person, wobei die Speichereinrichtung mit einer Empfangseinrichtung (8) zum Empfang der biometrischen Daten (BD) und einer Sendeeinrichtung (8) zum Übertragen der personenbezogenen Daten (PD) ausgebildet ist, wobei
die Speichereinrichtung Mittel zum Vergleich (9) der empfangenen biometrischen Daten (BD) mit darin hinterlegten, den personenbezogenen Daten (PD) zugeordneten biometrischen Datensätzen umfasst, und
die Speichereinrichtung Mittel zur Auswahl (9) desjenigen biometrischen Datensatzes umfasst, welcher mit den empfangenen biometrischen Daten (BD) die größte Übereinstimmung aufweist und bei welchem das Maß der Übereinstimmung oberhalb eines vorgegebenen Schwellenwertes liegt.

14. System nach Anspruch 13, **dadurch gekennzeichnet, daß** die Biometrieerfassungseinrichtung (2) eine optische Aufnahmeeinheit umfaßt.

15. System nach Anspruch 13 oder 14, **dadurch gekennzeichnet, daß** die Biometrieerfassungseinrichtung (2) eine kapazitive Aufnahmeeinheit umfaßt.

16. System nach einem der Ansprüche 13 bis 15, **dadurch gekennzeichnet, daß** die Biometrieerfassungseinrichtung (2) eine Beleuchtungseinrichtung umfaßt.

17. System nach einem der Ansprüche 13 bis 16, **gekennzeichnet durch** eine Analyseeinrichtung, welche die biometrischen Daten (PD) bezüglich bestimmter Parameter analysiert.

18. System nach einem der Ansprüche 13 bis 17, **gekennzeichnet durch** Mittel zum Komprimieren der biometrischen Daten (PD) vor dem Übersenden an eine Speichereinrichtung (6).

19. System nach einem der Ansprüche 13 bis 18, **gekennzeichnet durch** einen Encoder zu Codieren der biometrischen Daten (PD) und/oder einen Decoder zum Decodieren der personenbezogenen Daten (PD).

20. System nach einem der Ansprüche 13 bis 19, **gekennzeichnet durch** eine Mobilfunkeinrichtung (4).

21. System nach einem der Ansprüche 13 bis 20, **dadurch gekennzeichnet, daß** das Gerät ein mobiles Gerät (1) ist.

22. System nach Anspruch 21, **dadurch gekennzeichnet, daß** das Gerät ein Mobiltelefon (1) mit einer Biometrieerfassungseinrichtung (2) ist.

23. System nach einem der Ansprüche 13 bis 21, **dadurch gekennzeichnet, daß** die Biometrieerfassungseinrichtung eine Schnittstelle zum Anschluß an ein Mobiltelefon umfasst.

## Claims

1. Method for transferring personal data (PD), wherein a person's biometric data (BD) is recorded by a biometric acquisition device (2) and sent to a central storage device (6) for storing the personal data (PD), and on the basis of this biometric data (BD) personal data (PD) for that person is selected in the storage device (6) and said personal data (PD) is sent to a receiving device (4) located at the site of the biometric acquisition device (2) where it is further processed and/or output,
the received biometric data (BD) being compared in the storage device (6) with biometric data records stored there and assigned to the personal data (PD),
**characterised in that** the biometric data record having the closest match with the received biometric data (BD) and for which the degree of matching is above a predefined threshold is selected and the stored personal data (PD) assigned to said biometric data record is sent to the receiving device (4).

2. Method according to Claim 1, **characterised in that** the biometric acquisition device (2) and the receiving device (4) are implemented as mobile units.

3. Method according to Claim 1 or 2,**characterised in that** the biometric acquisition device (2) and the receiving device (4) are jointly disposed in a mobile unit (1).

4. Method according to one of Claims 1 to 3, **characterised in that** the personal data (PD) is medical data.

5. Method according to one of Claims 1 to 4, **characterised in that** the biometric data (BD) includes a fingerprint recording.

6. Method according to one of Claims 1 to 5, **characterised in that** the biometric data (BD) includes an iris pattern recording.

7. Method according to one of the preceding Claims, **characterised in that** the biometric data (BD) is analysed in respect of certain parameters, and the biometric data (PD) is used in the form of the thereby obtained parameter values to select the personal data in the storage device (6).

8. Method according to Claim 7, **characterised in that** the analysis of the biometric data (BD) in respect of certain parameters takes place in the biometric acquisition device (2), and the biometric data (BD) is sent in the form of the determined parameter values to the storage device (6).

9. Method according to one of the preceding Claims, **characterised in that**, in addition to the biometric data (BD), a scaling parameter of the biometric acquisition device (2) is sent to the storage device (6).

10. Method according to one of the preceding claims, **characterised in that** the biometric data (BD) and/or the personal data (PD) is compressed prior to transfer.

11. Method according to one of the preceding claims, **characterised in that** the biometric data (BD) and/or the personal data (PD) is transferred in encoded form.

12. Method according to one of the preceding claims, **characterised in that** the biometric data (BD) and/or the personal data (PD) is sent in packet format over a mobile communication channel.

13. System for carrying out the method according to one of the preceding claims, comprising
- a unit incorporating a biometric acquisition device (2) for recording a person's biometric data (BD), a transmitting device (4) for transferring the biometric data, a receiving device (4) for receiving the personal data (PD) and a device (3) for outputting and/or further processing said data (PD), and
- a storage device (6) for storing the person's personal data (PD), said storage device being implemented with a receiving device (8) for receiving the biometric data (BD) and a transmitting device (8) for transferring the personal data (PD),
the storage device having means of comparing (9) the received biometric data (BD) with biometric data records stored therein and assigned to the personal data (PD), and
the storage device having means of selecting (9) the biometric data record most closely matching the received biometric data (BD) and for which the degree of matching is above a predefined threshold value.

14. System according to Claim 13, **characterised in that** the biometric acquisition device (2) comprises an optical recording unit.

15. System according to Claim 13 or 14, **characterised in that** the biometric acquisition device (2) comprises a capacitive recording unit.

16. System according to one of Claims 13 to 15, **characterised in that** the biometric acquisition device (2) comprises an illumination device.

17. System according to one of Claims 13 to 16, **characterised by** an analysis device which analyses the biometric data (PD) in respect of certain parameters.

18. System according to one of Claims 13 to 17, **characterised by** means of compressing the biometric data (PD) prior to transfer to a storage device (6).

19. System according to one of Claims 13 to 18, **characterised by** an encoder for encoding the biometric data (PD) and/or a decoder for decoding the personal data (PD).

20. System according to one of Claims 13 to 19, **characterised by** a mobile communication device (4).

21. System according to one of Claims 13 to 20, **characterised in that** the unit is a mobile unit (1).

22. System according to Claim 21, **characterised in that** the unit is a mobile telephone (1) incorporating a biometric acquisition device (2).

23. System according to one of Claims 13 to 21, **characterised in that** the biometric acquisition device comprises an interface for connecting to a mobile telephone.

## Revendications

1. Procédé de transmission de données à caractère personnel (PD) dans lequel des données biométriques (BD) d'une personne sont saisies au moyen d'un dispositif de saisie biométrique (2) et envoyées à un dispositif central de mémoire (6) pour l'enregistrement des données à caractère personnel (PD), et dans lequel,
à l'aide de ces données biométriques (BD), des données à caractère personnel (PD) concernant cette personne sont sélectionnées dans le dispositif de mémoire (6) et dans lequel ces données à caractère personnel (PD) sont envoyées à un dispositif récepteur (4) situé à l'endroit où se trouve le dispositif de saisie biométrique (2), continuent d'y être traitées et/ou y sont émises, les données biométriques (BD) reçues étant comparées, dans le dispositif de mémoire (6), avec des jeux de données biométriques qui y sont déposés et sont associés aux données à caractère personnel (PD),
**caractérisé en ce que**
le jeu de données biométriques qui présente la plus grande coïncidence avec les données biométriques (BD) reçues et pour lequel la mesure de la coïncidence est supérieure à une valeur seuil donnée est sélectionné et **en ce que** les données à caractère personnel (PD) enregistrées et associées à ce jeu de données biométriques sont envoyées au dispositif récepteur (4).

2. Procédé selon la revendication 1, **caractérisé en ce que** le dispositif de saisie biométrique (2) et le dispositif récepteur (4) sont réalisés sous la forme d'appareils mobiles.

3. Procédé selon la revendication 1 ou 2, **caractérisé en ce que** le dispositif de saisie biométrique (2) et le dispositif récepteur (4) sont situés ensemble dans un appareil mobile (1).

4. Procédé selon l'une des revendications 1 à 3, **caractérisé en ce que** les données à caractère personnel (PD) sont des données médicales.

5. Procédé selon l'une des revendications 1 à 4, **caractérisé en ce que** les données biométriques (BD) comprennent une prise d'empreinte digitale.

6. Procédé selon l'une des revendications 1 à 5, **caractérisé en ce que** les données biométriques (BD) comprennent une prise du dessin de l'iris.

7. Procédé selon l'une des revendications précédentes, **caractérisé en ce que** les données biométriques (BD) sont analysées pour ce qui est de certains paramètres et **en ce que** les données biométriques (PD) sont utilisées sous la forme des valeurs paramétriques ainsi établies pour sélectionner les données à caractère personnel dans le dispositif de mémoire (6).

8. Procédé selon la revendication 7, **caractérisé en ce qu'**il est procédé à l'analyse des données biométriques (BD) pour ce qui est de certains paramètres dans le dispositif de saisie biométrique (2) et **en ce que** les données biométriques (BD) sont envoyées au dispositif de mémoire (6) sous la forme des valeurs paramétriques déterminées.

9. Procédé selon l'une des revendications précédentes, **caractérisé en ce qu'**un paramètre de mise à l'échelle est envoyé, en plus des données biométriques (BD), par le dispositif de saisie biométrique (2) au dispositif de mémoire (6).

10. Procédé selon l'une des revendications précédentes, **caractérisé en ce que** les données biométriques (BD) et/ou les données à caractère personnel (PD) sont comprimées avant la transmission.

11. Procédé selon l'une des revendications précédentes, **caractérisé en ce que** les données biométriques (BD) et/ou les données à caractère personnel (PD) sont transmises codées.

12. Procédé selon l'une des revendications précédentes, **caractérisé en ce que** les données biométriques (BD) et/ou les données à caractère personnel (PD) sont émises par paquets sur un canal radio mobile.

13. Système permettant d'exécuter le procédé selon l'une des revendications précédentes, comprenant
- un appareil avec un dispositif de saisie biométrique (2) pour la saisie de données biométriques (BD) d'une personne, un dispositif émetteur (4) pour la transmission des données biométriques, un dispositif récepteur (4) pour la réception des données à caractère personnel (PD) et un dispositif (3) pour l'émission et/ou la poursuite du traitement de ces données (PD) et
- un dispositif de mémoire (6) pour l'enregistrement des données à caractère personnel (PD) de la personne, le dispositif de mémoire étant réalisé tel qu'il comporte un dispositif récepteur (8) pour la réception des données biométriques (BD) et un dispositif émetteur (8) pour la transmission des données à caractère personnel (PD),
le dispositif de mémoire comprenant des moyens de comparaison (9) des données biométriques (BD) reçues avec des jeux de données biométriques qui y sont déposés et sont associés aux données à caractère personnel (PD) et
le dispositif de mémoire comprenant des moyens de sélection (9) du jeu de données biométriques qui présente la plus grande coïncidence avec les données biométriques (BD) reçues et dans lequel la mesure de la coïncidence est supérieure à une valeur seuil donnée.

14. Système selon la revendication 13, **caractérisé en ce que** le dispositif de saisie biométrique (2) comprend une unité de prise optique.

15. Système selon la revendication 13 ou 14, **caractérisé en ce que** le dispositif de saisie biométrique (2) comprend une unité de prise capacitive.

16. Système selon l'une des revendications 13 à 15, **caractérisé en ce que** le dispositif de saisie biométrique (2) comprend un dispositif d'éclairage.

17. Système selon l'une des revendications 13 à 16, **caractérisé par** un dispositif d'analyse qui analyse les données biométriques (PD) pour ce qui est de certains paramètres.

18. Système selon l'une des revendications 13 à 17, **caractérisé par** des moyens de compression des données biométriques (PD) avant la transmission à un dispositif de mémoire (6).

19. Système selon l'une des revendications 13 à 18, **caractérisé par** un encodeur pour coder les données biométriques (PD) et/ou un décodeur pour décoder les données à caractère personnel (PD).

20. Système selon l'une des revendications 13 à 19, **caractérisé par** un dispositif radio mobile (4).

21. Système selon l'une des revendications 13 à 20, **caractérisé en ce que** l'appareil est un appareil mobile (1).

22. Système selon la revendication 21, **caractérisé en ce que** l'appareil est un téléphone mobile (1) avec un dispositif de saisie biométrique (2).

23. Système selon l'une des revendications 13 à 21, **caractérisé en ce que** le dispositif de saisie biométrique comprend une interface de connexion à un téléphone mobile.
